# FASCICULE DE BREVET EUROPEEN

(11) **EP 0 680 757 B1**
(45) Date de publication et mention de la délivrance du brevet: **13.12.2000**
(21) Numéro de dépôt: 95401037.7
(22) Date de dépôt: 04.05.1995
(51) Int. Cl.: A61K 31/195

(54) **Utilisation de principes actifs protégés vis-à-vis de la dégradation dans le rumen comme hépatoprotecteurs**
Verwendung von gegen Abbau im Pancen von Wiederkäuern geschützten Wirkstoffen als Hepatoprotektor
Use of active substances protected against degradation in the rumen of ruminants as hepatoprotector

(30) Priorité: 06.05.1994 FR 9405617
(43) Date de publication de la demande: 08.11.1995
(73) Titulaire: RHONE-POULENC NUTRITION ANIMALE, 92160 Antony (FR)
(72) Inventeur: Bauchart, Dominique, F-63960 Veyre-Monton (FR); Chilliard, Yves, F-63122 Ceyrat (FR); Durand, Denys, F-63110 Beaumont (FR); Gruffat, Dominique, F-63000 Clermont-Ferrand (FR); Ollier, Alain, F-63122 Saint Genes Champanelle (FR); Robert, Jean-Claude, F-03310 Neris Les Bains (FR); Williams, Peter, F-78150 Le Chesnay (FR)
(74) Mandataire: Brachotte, Charles

(56) Documents cités:
- EP-A- 0 260 186
- EP-A- 0 321 337
- EP-A- 0 406 041
- FR-A- 2 524 269
- CHEMICAL ABSTRACTS, vol. 106, no. 11, 16 Mars 1987 Columbus, Ohio, US; abstract no. 83423, 'POSITIVE EFFECTS OF THE METHIONINE (BYPASS) AND CHOLINE (BYPASS) COMBINATION IN THE NUTRITION OF RUMINANTS'
- JOURNAL OF DAIRY SCIENCE, 1992 page 279 D. DURAND ET AL. 'EFFECTS OF LYSINE AND METHIONINE ON IN VIVO HEPATIC SECRETION OF VLDL IN THE HIGH HIELDING DAIRY COW'
- VETERINARY CLINICS OF NORTH AMERICA: FOOD ANIMAL PRACTICE, vol. 4, no. 2, 1988 pages 269-287, T.H. HERDT 'FATTY LIVER IN DAIRY COWS'
- THE VETERINARY CLINICS OF NORTH AMERICA, vol. 4, no. 2, 1988 pages 379-390, B.J. GERLOFF 'FEEDING THE DRY COW TO AVOID METABOLIC DISEASE'
- CHEMICAL ABSTRACTS, vol. 91, no. 9, 27 Août 1979 Columbus, Ohio, US; abstract no. 73444, D. STOIKOV 'ORAL ADMINISTRATION OF DL-METHIONINE IN THE PROPHYLAXIS AND TREATMENT OF KETOSIS IN HIGHLY-PRODUCTIVE COWS'
- DIABETE & METABOLISM, vol. 18, no. 1BIS, 1992 pages 145-149, A. MAZUR ET AL. 'LIPOPROTEIN METABOLISM IN FATTY LIVER DAIRY COWS'

## Description

La présente invention concerne l'utilisation d'amino acides protégés vis-à-vis de la dégradation dans le rumen chez le ruminant et surtout leur effet sur son état hépatique en début de lactation.

Il est connu d'après l'article de Remond et al (Ann. Zootech., 1989, 38, 129-137) qu'une méthionine protégée donnée à des vaches en début de lactation permet d'obtenir une augmentation de la quantité de lait produite au cours des deux premières semaines après la mise bas de 2,6 et 1,2 kg/jr et une augmentation moyenne des protéines d'environ 27 g par jour ce qui se traduit par une augmentation du taux protéique moyen du lait de 1 à 2%.

Il est aussi connu selon l'article de Chilliard et al. (Reprod. Nutr. Develop., 1987, 27 (2A), 327-398) qu'en début de lactation, la capacité d'ingestion des vaches laitières est affaiblie; la vache laitière se trouve en déficit énergétique et pour le combler, elle mobilise ses réserves corporelles. Les triglycérides stockés dans les tissus adipeux sont hydrolysés en acide gras et transportés jusqu'au foie, dans lequel ils sont transformés en triglycérides et en corps cétoniques. La capacité de recyclage et d'exportation de ces acides gras non estérifiés par le foie sous forme de triglycérides est faible car ils doivent être transportés par l'intermédiaire de lipoprotéines telles que les VLDL (lipoprotéines de très faible densité). Or, il est connu selon l'article de Rayssiguier et al (Res. Vet. Sci., 1988, 45, 389-393) qu'en début de lactation les VLDL sont synthétisées en très faible quantité ce qui entraîne un surstockage de graisse au niveau hépatique et par la même se traduit parfois par une stéatose hépatique Cette faible synthèse de VLDL serait due à une disponibilité insuffisante d'un des constituants lipidiques (cholestérol, phospholipides) ou protéique (apoprotéine B)

Le document Krmiva, Vol. 28 (5-6), 1986, pages 139-142: G.Panciroli décrit l'alimentation de bovins au moyen d'un mélange de choline et de méthionine. L'administration de choline prévient l'apparition de la stéatose du foie et, si la stéatose est installée, son administration permet d'éliminer les graisses et de traiter cette maladie.

Le document Journal of Dairy Science, 1992, page 279; D. Durand et al décrit l'utilisation d'un mélange de lysine et de méthionine permettant d'augmenter la sécrétion de VLDL. Ce document suggère, en outre au vu de ces résultats, que l'infiltration lipidique hépatique pourrait être réduite chez les ruminants en lactation. Le document The Veterinary Clinics of North America; Vol.4(2), 1988, pages 379-390; B.J Gerloff et al suggère l'utilisation de niacine et d'acide nicotinique pour réduire l'utilisation excessive de graisses par mobilisation interne et pour ainsi réduire la cétose et la stéatose hépatique.

Le document. Vet.-Med. Nauki, Vol.15(7) 1978, pages 92-98; D. Stoikov décrit l'administration orale de DL-méthionine pour la prophylaxie et le traitement de la cétose. La méthionine étant digéré dans le rumen, ce composé ne peut en conséquence se retrouver dans le sang et ne peut être efficace pour la prévention ou le traitement de la stéatose.

Il est apparu de façon tout à fait surprenante que chez les ruminants l'ingestion de méthionine et/ou de lysine protégés vis-a-vis de la dégradation dans le rumen et libérant lesdits principes actifs dans la caillette et/ou l'intestin permettait de réduire ou de prévenir la stéatose hépatique

Cette réduction de la stéatose hépatique est due d'une part à une augmentation de la libération des lipoprotéines hépatiques qui se traduit par l'augmentation de l'expression du gène hépatique de l'apoprotéine B (augmentation de l'ARNm de l'apoB) et de la teneur intrahépatique de l'apo B; en effet, l'apoprotéine, en tant qu'élément structural indispensable à la synthèse de VLDL, est l'un des facteurs majeurs limitants de la production de particules riches en triglycérides.

Parmi la méthionine et/ou la lysine protégée on préfère utiliser la méthionine protégée.

Parmi les composés permettant une protection efficace dans le milieu du rumen et une libération dans la caillette ou l'intestin grêle on peut citer deux grands types de composés. Certains de ces composés permettent une protection et une libération qui est provoquée uniquement par un phénomène chimique, due à la différence de la valeur du pH entre le rumen (5-6) et la caillette (2-3); on peut citer dans cette catégorie d'abord les polymères pH sensibles tels que les copolymères du styrène et de la vinylpyridine en mélange avec des corps gras tels que les acides gras, les huiles végétales ou animales hydrogénées De nombreux brevets tels que les brevets US 4 877 621 ou US 4 832 967 décrivent des compositions contenant de tels composés. Ces compositions permettent une excellente protection in vivo et in vitro des substances nutritives ou médicamenteuses et une bonne libération des principes actifs. Elles présentent le seul inconvénient de contenir un produit chimique de synthèse qui d'un point de vue alimentaire et écologique n'est pas toujours très apprécié tant du point de vue des autorités administratives d'autorisation de mise sur le marché que de celui des consommateurs.

Dans cette première catégorie de compositions où la libération de l'acide aminé est provoquée uniquement par un phénomène chimique, on peut citer, aussi les compositions à base de chitosane et d'acides carboxyliques. Ces compositions sont par exemple décrites dans le brevet français publie sous le numéro 2 524 269. Ces compositions contiennent une quantité de principes actifs toujours inférieure à 60% en poids et plus préférentiellement de l'ordre de 30% o pour permettre une protection au niveau du rumen de l'ordre de 80 à 90% sur 24 heures et contiennent, en outre, une ou plusieurs charges minérales pH sensibles, en quantité notable (10 à 20% en poids).

Une deuxième catégorie de brevets décrit des compositions utilisant les propriétés d'hydrolyse enzymatique de certains composés naturels tels que la zéine. Parmi ces brevets on peut citer le brevet US 4 983 403 ou la demande de brevet européen EP 0 406 041.

Selon une variante, le principe actif est protégé par un mélange à base de chitosane.

Selon une autre variante, le principe actif est protégé par un polymère naturel dont la libération est provoquée par une hydrolyse enzymatique, de préférence un polymère à base de zéine.

La présente invention décrit l'effet de méthionine et/ou lysine, protégés vis à vis de la dégradation dans le rumen et libérables dans la caillette ou l'intestin grêle, sur la libération des lipoprotéines hépatiques riches en triglycérides ainsi que l'effet sur la synthèse de l'ARN m de l'apoprotéine B et sur la libération d'apoprotéine B au niveau hépatique.

La présente invention sera plus complètement décrite à l'aide des exemples suivants.

### EXEMPLE 1

L'objectif du présent essai est d'évaluer dans quelle mesure une supplémentation en méthionine protégée contre les fermentations du rumen préparée notamment selon le brevet US 4 877 621 peut limiter la stéatose hépatique et la cétose chez des vaches grasses en début de lactation recevant une ration présentant une faible concentration énergétique.

### Matériel et méthodes

### 1 - Animaux

Quinze vaches sont réparties en 2 lots équilibrés sur la date de vêlage, la note d'état corporel, le poids vif 3 mois avant vêlage et la production laitière maximum de la lactation précédente.

Les deux lots sont les suivants : témoin sans méthionine (T, 8 vaches), lot recevant de la méthionine protégée (M, 7 vaches), une ayant dû être sortie de l'expérience au début de la partie post expérience (42 jours après le vêlage).

### 2- Alimentation

### a) Fin de gestation

Les animaux reçoivent de l'ensilage de maïs à volonté, avec du concentré selon la protocole suivant, S(-4) à S(-1) indiquant les semaines avant le vêlage:

| kg | S(-4) et S(-3) | S(-2) | S(-1) |
|---|---|---|---|
| Concentré | 0,5 | 1,5 | 2,5 |
| Tourteaux soja tanné | 1 | 1,0 | 1,0 |
| Tourtaux soja non tanné | 0,5 | 0,5 | 0,5 |

L'apport de calcium est limité (100 g/j puis suppression 3 semaines avant vêlage) et on distribue un supplément d'environ 1 kg de foin par jour.

### b) Début de la lactation

Les animaux reçoivent, en un repas par jour, une ration complète de composition constante pendant les 3 premiers mois de lactation, contenant 75 % d'ensilage de maïs, 20 % de tourteau de soja tanné et 5 % de tourteau de soja non tanné. On rajoute du concentré minéral vitaminé (200 g/j), et du foin (1 kg/j).

### Méthionine protégée (22 g/j)

Distribuée le matin à jeun dans un seau avec 200 g de tourteau non tanné, pendant 5 jours à toutes les vaches (15) trois à quatre semaines avant mise-bas, puis tous les jours aux vaches du lot M à partir du 3 ou 5ème jour après la mise-bas (au jour 3 à 5 de lactation), jusqu'en 6ème semaine de lactation incluse (42 jours).

### Dosage des lipoprotéines

### - Séparation des classes majeures de lipoprotéines

Les VLDL (lipoprotéines de très basse densité) ont été séparées à partir de 20 ml de plasma par ultracentrifugation de flottaison : 40 000 tr/min pendant 16 heures et à 15°C.

Les IDL, LDL, HDLI et HDLh (lipoprotéines de densité intermédiaire, basse, haute densité légère et haute densité lourde) ont été séparées par gradient de densité à 39 000 tr/min pendant 46 heures et à 15°C, selon la technique décrite par Chapman, Golstein Lagrange et Laplaud, J. Lip. Res., 22, 339-358 (1981) et adaptée par Bauchard et al. (1989, J. Lip. Res., 30, 1499-1514).

Nous avons obtenu ainsi 5 classes de lipoprotéines :
- VLDL : d<1,018 g/ml
- IDL : 1,018 g/ml <d< 1,026 g/ml
- LDL: 1,026 g/ml <d< 1,060 g/ml
- HDLI : 1,060 g/ml <d< 1,091 g/ml
- HDLh : 1,091 g/ml <d< 1,180 g/ml

Ces différentes fractions lipoprotéiques ont été ensuite dialysées à 4°C pendant 7 heures avec un système de microdialyse afin d'éliminer le KBr.

### - Séparation des LDL vraies et des HDL very light (HDLvl) dans la zone de densité 1,040-1,090 g/ml

Nous avons dans un premier temps vérifié l'hétérogénéité de taille des particules dans la zone de densité 1,040-1,090 g/ml par électrophorèse sur gel en gradient d'acrylamide (gel PAA : 2,5 - 16 %) de tous nos animaux à chacun des différents stades étudiés. Après s'être assuré de l'homogénéité de nos animaux, nous avons regroupé les plasmas par stade et par traitement puis isolé les particules lipoprotéiques par ultracentrifugation de flottaison selon le protocole décrit par Laplaud et al. (J. Lip. Res., 1991, 32, 1429-1439), dans la zone de densité à 1,040-1,090 g/ml. Ces lipoprotéines de type HDL et LDL ont été ensuite séparées par chromatographie d'affinité sur colonne d'héparine-Sépharose ce qui nous a permis de séparer et quantifier les particules LDL (sans contamination par des HDL) appelées "LDL vraies" d'une part et des HDL non contaminées par des LDL d'autre part. Ces particules ont ensuite été analysées, comme les autres classes lipoprotéiques séparées sur gradient, notamment la composition chimique.

### - Détermination de la composition chimique

Sur chacune des fractions lipoprotéiques plasmatiques comme sur les plasmas totaux, nous avons dosé les apoprotéines A-I et B par immunodiffusion radiale selon la méthode de Mancini adaptée par Auboiron et al., Reprod. Nutr. Develop., 2, 2275 (1989). Les protéines totales des fractions lipoprotéiques ont été dosées par colorimétrie par la méthode colorimétrique utilisant le réactif à base d'acide bicinchoninic (BCA, Pierce, IL, USA).

### - Dosage de l'ARNm de l'apo B hépatique

Les ARN totaux sont isolés à partir des biopsies de foie (environ 200 mg) par le thiocyanate de guanidine/phénol-chloroforme d'après la méthode décrite par Chomczynski et Sacchi, Ann. Biochem., 162, 156-159 (1987).

La quantification de l'ARNm d'apo B est réalisée selon la méthode suivante : 70 g de chaque extrait d'ARN totaux sont dénaturés en présence de formaldéhyde et déposés par dilutions successives de 1/2 en 1/2 sur une membrane de nitrocellulose en utilisant un appareil de Dot-Blot (Schleicher & Schuell, SRC 26 D Minifold 1) soit 4 dépôts de 30, 15, 7,5 et 3,75 µg environ pour chaque extrait d'ARN. L'hydridation est réalisée avec une sonde synthétisée à partir d'un fragment d'ADNc de l'apo B humaine putifiée en laboratoire et marquée au (α³²P) dCTP (desoxycytidyltriphosphate) par marquage aléatoire (random priming). L'abondance de l'ARNm d'apo B est calculée par analyse densitométrique d'autoradiographies obtenues après exposition des membranes pendant 16 heures à -80°C.

La comparaison entre les différentes autoradiographies étant impossible, nous avons choisi de déposer sur une même membrane les ARN totaux correspondant aux différents stades de prélèvement (S1, S2, S4 et S12) de 5 vaches (2 témoins et 3 méthionines ou vice-versa) Ceci nous a permis de déterminer l'évolution des teneurs en ARNm d'apo B au cours du début de lactation et de comparer cette évolution entre les vaches des lots témoin et expérimentaux.

### - Dosage de l'apoprotéine B intrahépatique

Les protéines totales intrahépatiques ont été extraites par homogénéisation de fragments de foie prélevés par biopsie (50 mg) dans une solution de lyse hautement concentrée en détergents (désoxycholate de sodium, Triton X100) et en inhibiteurs de protéases (benzamidine, PMSF, ou phényl méthyl sulfonyl fluoride, leupeptine, pepstatine)

Les protéines intrahépatiques totales extraites ont été quantifiées par dosage colorimétrique selon la méthode de Bradford (1976) Des quantités comparables (100 µg) de protéines totales ont été déposées sur gradient de gel de polyacrylamide (3-7,5%) en conditions dénaturantes Après migration électrophorétique, les protéines sont transférées sur membrane de nitrocellulose par électrotransfert. Ces membranes sont incubées en présence d'anticorps polyclonal de lapin anti-apo B bovine La fixation de l'anticorps sur la bande correspondant à l'apo B est révélée en incubant la membrane en présence de protéine A marquée a l'iode 125 Les teneurs en apo B intrahépatiques sont calculées par analyse densitométrique à partir des autoradiographies obtenues après exposition des membranes pendant 16h à -80°C.

### 3 - Lipides et apoprotéines plasmatiques (Tableau 1)

Dès la première semaine de lactation, la concentration en acides gras non estérifiés circulants est très élevée et elle le reste au stade S2 ; ces taux vont ensuite diminuer de près de 50 % (P < 0,05) de S2 à S4 (en période post-prandiale), pour atteindre des valeurs très faibles en S12. Les teneurs en cholestérol libre, en esters de cholestérol et phospholipides du plasma subissent une évolution inverse, voyant leur taux augmenter fortement entre S1 et S12, ces taux étant multipliés par des facteurs 5, 3 et 2 respectivement. Par contre, la triglycéridémie reste constante tout au cours de cette période. Aucune différence significative n'est observée entre les 2 lots pour l'ensemble des fractions lipidiques étudiées.

Le taux d'apoprotéine B totale plasmatique n'évolue pas entre S1 et S2 et reste proche de 4 mg/dl, puis augmente jusqu'à une valeur de 11 mg/dl au stade S12. Cette évolution est comparable pour les 2 lots (témoin et méthionine).

Le taux d'apoprotéine A-I totale plasmatique augmente, comme dans le cas de l'apo B, principalement entre les stades S2 et S12 atteignant en moyenne 100 mg/dl en S12. Par contre, une légère différence est observée dans la forme d'évolution entre nos 2 lots. En effet, l'augmentation du taux d'apo A-I est essentiellement observée entre les stades S2 et S4 pour le lot "méthionine" alors que cette augmentation est beaucoup plus progressive dans le lot "témoin". Malgré cette différence de forme d'évolution, aucune différence significative de leur teneur plamatique est observée entre les 2 lots, quel que soit le stade de lactation étudié.

**TABLEAU 1**

| | | Semaines après vêlage | | | |
|---|---|---|---|---|---|
| Composés plasmatiques | | S1 | S2 | S4 | S12 |
| AGNE | T | 28.6 ± 3.8^{a} | 26.7 ± 5.1^{a} | 13.2 ± 2.2^{b} | 2.8 ± 0.8^{c} |
| | M | 26.1 ± 4.4^{a} | 22.8 ± 2.8^{a} | 16.2 ± 2.6^{b} | 2.3 ± 0.6^{c} |
| CL | T | 3.3 ± 0.5^{a} | 5.0 ± 1.1^{a} | 9.7 ± 2.1^{b} | 16.5 ± 2.1^{c} |
| | M | 4.3 ± 0.8^{a} | 5.2 ± 1.2^{a} | 10.7 ± 2.7^{b} | 16.8 ± 2.1^{c} |
| EC | T | 75.9 ± 5.1^{a} | 105.7 ± 9.4^{b} | 156.8 ± 13.3^{c} | 205.2 ± 11.9^{d} |
| | M | 72.8 ± 6.7^{a} | 97.6 ± 6.0^{b} | 160.7 ± 9.1^{c} | 213.7 ± 7.6^{d} |
| PL | T | 86.8 ± 4.1^{a} | 120.5 ± 13.1^{b} | 143.5 ± 12.1^{b} | 154.1 ± 12.5^{b} |
| | M | 85.3 ± 5.5^{a} | 121.3 ± 23.8^{a} | 132.6 ± 15.5^{a} | 187.9 ± 13.5^{b} |
| TG | T | 17.8 ± 1.9 | 17.8 ± 1.2 | 16.6 ± 0.8 | 17.5 ± 1.0 |
| | M | 16.4 ± 1.2 | 16.5 ± 1.0 | 19.8 ± 3.0 | 17.3 ± 0.6 |
| apo B | T | 3.8 ± 1.0^{a} | 4.7 ± 1.0^{a} | 6.8 ± 1.9^{b} | 10.7 ± 2.4^{c} |
| | M | 3.7 ± 1.5^{a} | 3.7 ± 1.3^{a} | 4.8 ± 1.7^{a} | 11.2 ± 3.1^{b} |
| Apo A-I | T | 50.4 ± 7.1^{a} | 63.1 ± 8.3^{b} | 80.1 ± 15.3^{c} | 100.7 ± 16.8^{d} |
| | M | 70.6 ± 10.9^{a} | 69.1 ± 5.1^{a} | 100.8 ± 14.5^{b} | 106.9 ± 5.9^{b} |

Tableau: Evolution des teneurs en lipides (AGNE, Acides Gras Non Estérifiés ; CL, Cholestérol Libre ; EC, Esters de Cholestérol ; PL, Phospholipides; ; TG : Triglycérides) et en apoprotéines A-I et B (Apo A-I, Apo B) plasmatiques (mg/dl ; moyenne ± ES) chez la vache laitière au cours du début de lactation recevant un régime à base d'ensilage de maïs (régime témoin T, n=8) ou le même régime supplémenté en méthionine protégée de 4 à 6 jours jusqu'à 42 jours après le vêlage (régime méthionine M, n=7); S1=3-5 j; S2=10-12 j; S4=24-26 j; S12=71-89 j après le vêlage.

Les concentrations plasmatiques des lipides ont été comparées à l'intérieur d'un même lot par test "t" dans le cas de séries appariées; les valeurs affectées de 2 lettres différentes sont significativement différentes au seuil de 5%.

### 4 - Lipoprotéines plasmatiques

L'évolution globale des différentes familles lipoprotéiques montre que les concentrations plasmatiques en VLDL, IDL et HDL lourdes (HDLh) sont peu modifiées au cours des 12 premières semaines de lactation. Par contre, les taux plasmatiques en LDL et de HDL légères (HDL1) augmentent très fortement entre S1 et S12 (tableau 2). La composition lipidique de ces deux familles de lipoprotéines n'est que peu modifiée.

**TABLEAU 2**

| | | Semaines après vêlage | | | |
|---|---|---|---|---|---|
| | | S1 | S2 | S4 | S12 |
| LDL "vraies" | T | 21.4 ± 3.4^{a} | 22.8 ± 3.1^{a} | 34.9 ± 5.5^{b} | 63.0 ± 5.8^{c} |
| | M | 14.6 ± 2.9^{a} | 18.2 ± 4.0^{a} | 24.0 ± 5.9^{b} | 59.3 ± 6.4^{c} |
| HDLvl | T | 6.8 ± 5.5^{ab} | 0.3 ± 2.7^{a} | 17.4 ± 8.2^{b} | 71.0 ± 19.0^{c} |
| | M | 3.5 ± 4.1^{a} | 5.4 ± 2.7^{ab} | 27.4 ± 10.3^{b} | 98.0 ± 38.4^{c} |

Evolution de la concentration en lipoprotéine de type LDL "vraies" et HDL très légères (HDLvl) (mg/dl; moyenne± ES) isolées par ultracentrifugation en gradient de densité puis par chromatographie d'affinité sur héparine-sépharose, chez la vache laitière au cours du début de lactation recevant un régime à base d'ensilage de maïs (régime témoin T, n=8) ou le même régime supplémenté en méthionine protégée (régime méthionine M, n=7) de 4 à 6 jours jusqu'à 42 jours après le velage.

Les concentrations plasmatiques des lipides ont été comparées à l'intérieur d'un même lot par test "t" dans le cas de séries appariées; les valeurs affectées de 2 lettres différentes sont significativement différentes au seuil de 5%.

En revanche, les teneurs respectives en apoprotéines A-I et B des LDL isolées (tableau 3) entre les densités 1,020-1,060 g/ml varient fortement avec le stade de lactation étudié. Ainsi, la concentration en apo B augmente de 3 à 9 mg/dl entre S1 et S12 et celle de l'apo A-I de 2 à 16 mg/dl. La contribution de l'apoprotéine A-I au contenu total des particules dans cette zone s'élevant de 7 à 10 % et est plus rapide dans le temps pour le groupe recevant de la méthionine.

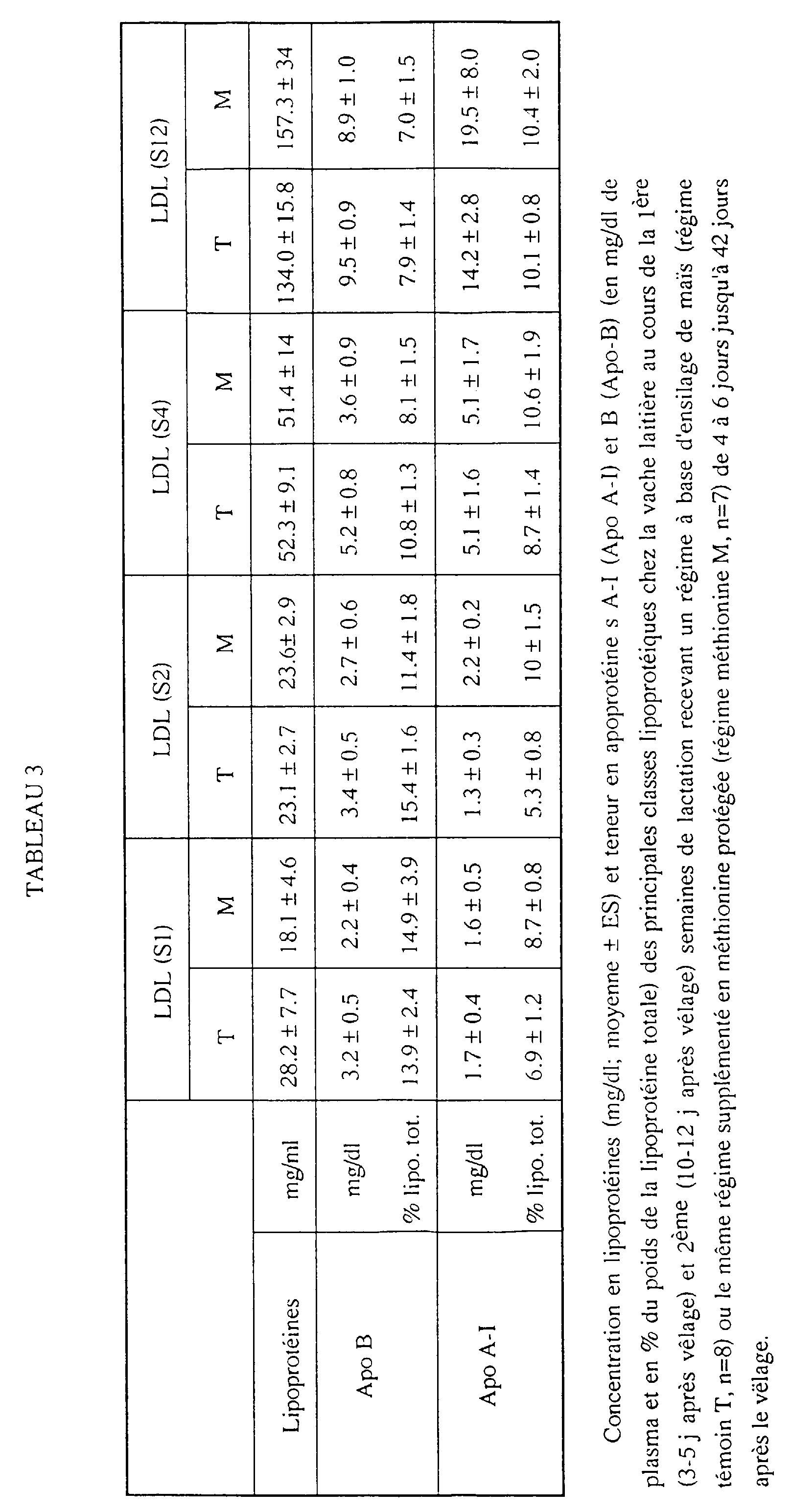

### 5 - Niveaux hépatiques en ARNm d'apo B (Tableaux 4 et 5)

Les résultats présentés concernent les 8 vaches du lot témoin, et les 6 ou 7 vaches du lot méthionine (7 en période de traitement avec la méthionine protégée et 6 sur la totalité de l'essai).

La quantification de l'ARNm d'apo B hépatique a été réalisée par analyse en Dot-Blot. Les évolutions des niveaux d'ARNm de l'apo B (unités arbitraires/µg d'ARN) entre 2 stades consécutifs de prélèvement (S2-S1; S4-S2 ; S12-S4) ont été calculées pour les 2 lots de vaches (tableau 4). Les importantes variations de niveaux d'ARNm d'apo B entre animaux et les différences d'intensité de signal existant entre autoradiographies induisent l'apparition d'écarts-types importants lors de ce calcul. Nous pouvons toutefois noter une augmentation significative des niveaux d'ARNm d'apo B intrahépatiques entre les stades S1 et S2 pour le lot méthionine (6 vaches sur 7, P < 0,035 ou 6 vaches sur 6, P < 0,02) et entre les stades S2 et S4 pour le lot témoin (7 vaches sur 8, P < 0,06).

**TABLEAU 4**

| | Evolution des teneurs hépatiques en ARNm d'apo B | | |
|---|---|---|---|
| Lots | S2-S1 | S4-S2 | S12-S4 |
| Témoin (n = 8) | 910 ± 607 | 1359 ± 698* | 658 ± 974 |
| Méthionine (n = 7) | 3505 ± 920** | 183 ± 1407 | - |
| Méthionine (n = 6) | 4109 ± 778*** | 263 ± 1454 | 1428 ± 1358 |

Evolution des teneurs intrahépatiques de l'ARNm d'apo B (unités arbitraires/µg d'ARN totaux, ± SE) entre 2 stades consécutifs de prélèvement chez la vache laitière en début de lactation recevant un régime à base d'ensilage de maïs (régime témoin T, n=8) ou le même régime supplémenté en méthionine protégée (régime méthionine M, n=7 ou n=6) de 3 à 5 jours jusqu'à 42 jours après le vêlage (S1=3-5 j; S2= 10-12 j; S4=24-26 j; S12=71-89 j).

Les valeurs sont significativement différentes de 0 au seuil de 6 % (*), 3,5 % (**) ou 2 % (***) (Test du signe).

Dans le but d'éliminer les écarts induits par les différences d'intensité de signal existant entre les autoradiographies, le rapport des niveaux hépatiques d'ARNm d'apo B mesurés entre 2 semaines consécutives de prélèvement (S2/S1 ; S4/S2 ; S12/S4) a été calculé (tableau 5). Ce rapport permet de mettre en évidence une augmentation significative (P < 0,01) de l'ARNm d'apo B de 50 % entre les stades S1 et S2 dans le lot méthionine alors qu'aucune variation significative n'est observée pour le lot témoin pour cette même période.

**TABLEAU 5**

| | Rapport des niveaux hépatiques d'ARNm d'apo B | | |
|---|---|---|---|
| Lots | S2/S1 | S4/S2 | S12/S4 |
| Témoin (n = 8) | 1.08 ± 0.07^{a} | 1.20 ± 0.10 | 1.10 ± 0.09 |
| Méthionine (n = 7) | 1.50 ± 0.10^{b} | 0.96 ± 0.12 | - |
| Méthionine (n = 6) | 1.58 ± 0.05^{b} | 0.98 ± 0.13 | 1.27 ± 0.17 |

Rapport des teneurs intrahépatiques de l'ARNm d'apo B (unités arbitraires/µg d'ARN totaux, ± SE) entre 2 stades consécutifs de prélèvement chez la vache laitière en début de lactation recevant un régime à base d'ensilage de maïs (régime témoin T, n=8) ou le même régime supplémenté en méthionine protégée (régime méthionine M, n=7 ou n=6) de 3 à 5 jours jusqu'à 42 jours après le vêlage (S1=3-5 j; S2= 10-12 j; S4=24-26 j; S12=71-89 j).

Les valeurs affectées de deux lettres différentes sont significativement différentes au seuil de 1%

### 6 - Variation des teneurs hépatiques d'apo B (Tableaux 6 et 7)

La quantification de l'apo B intrahépatique a été réalisée par analyse en Western-Blot. Les évolutions des niveaux de l'apo B (unités arbitraires / 10⁹ cellules) entre 2 stades consécutifs de prélèvement (S2-S1; S4-S2 ; S12-S4) ont été calculées pour les 2 lots de vaches (tableau 6). Les importantes variations de niveaux d'apo B entre les animaux et les différences d'intensité de signal existant entre autoradiographies induisent l'apparition d'écarts-types importants lors de ce calcul. Nous pouvons toutefois noter une augmentation significative des niveaux d'apo B intrahépatiques (d'après le test du signe) entre les stades S2 et S1 pour le lot méthionine (p < 0,01) et entre les stades S4 et S2 pour le lot témoin (p < 0,035).

**TABLEAU 6**

| | Evolution des teneurs hépatiques en apo B | | |
|---|---|---|---|
| Lots | S2-S1 | S4-S2 | S12-S4 |
| Témoin (n = 8) | -0,56 ± 6,9 | 19,10 ± 5,4* | 89,2 ± 21** |
| Méthionine (n = 7) | 18,20 ± 2,9** | 7,67 ± 7,2 | - |
| Méthionine (n = 6) | 14,00 ± 3,6** | 3,92 ± 7,1 | 76,5 ± 23** |

Evolution des teneurs intrahépatiques d'apo B (unités arbitraires/10⁹ cellules, ±SE ) entre 2 stades consécutifs de prélèvement chez la vache laitière au cours du début de lactation recevant un régime à base d'ensilage de maïs (régime témoin T, n=8) ou le même régime supplémenté en méthionine protégée (régime méthionine M, n=7 ou n=6) de 3 à 5 jours jusqu'à 42 jours après le vêlage (S1=3-5 j; S2=10-12 j; S4=24-26 j; S12=71-89 j).

Les différences sont significativement différentes de 0 au seuil de 3,5 % (∗) ou au seuil de 1 % (∗∗) (test U).

Dans le but d'éliminer les différences d'intensité de signal existant entre autoradiographies, le rapport des niveaux hépatiques d'apo B entre 2 semaines consécutives de prélèvement (S2/S1; S4/S2; S12/S4) a été calculé (tableau 7). Ce rapport permet de mettre en évidence une augmentation significative (p < 0,01) du niveau d'apo B de 68 % entre les stades S2 et S1 dans le lot méthionine alors qu'aucune variation significative n'est observée pour le lot témoin pour cette même période.

**TABLEAU 7**

| | Rapport des niveaux hépatiques d'apo B | | |
|---|---|---|---|
| Lots | S2/S1 | S4/S2 | S12/S4 |
| Témoin (n = 8) | 1.07 ± 0.12^{a} | 1.52 ± 0.20 | 2.49 ± 0.40 |
| Méthionine (n = 7) | 1.68 ± 0.09^{b} | 1.22 ± 0.16 | - |
| Méthionine (n = 6) | 1.66 ± 0.10^{b} | 1.14 ± 0.16 | 2.70 ± 0.54 |

Rapport des teneurs intrahépatiques d'apo B (unités arbitraires/µg d'ARN totaux, ±SE ) entre 2 stades consécutifs de prélèvement chez la vache laitière au cours du début de lactation recevant un régime à base d'ensilage de maïs (régime témoin T, n=8) ou le même régime supplémenté en méthionine protégée (régime méthionine M, n=7 ou n=6) de 3 à 5 jours jusqu'à 42 jours après le vêlage (S1=3-5 j; S2=10-12 j; S4=24-26 j; S12=71-89 j).

Les valeurs affectées de deux lettres différentes sont significativement différentes au seuil de 1%.

### EXEMPLE 2

On reproduit l'exemple 1 sur 16 vaches (6 témoins et 10 expérimentées). les vaches reçoivent la même alimentation de base que dans l'exemple 1 et reçoivent pour les vaches (M) 40 g de méthionine protégée (environ 28 g de méthionine digestible) et pour les vaches (M + L) 18 g de méthionine protégée (13 g de méthionine digestible) et 100 g de méthionine/lysine protégée (50 g de lysine digestible et 15 g de méthionine digestible).

Les résultats concernant les analyses sanguines sont indiquées dans le tableau 8, les résultats concernant les teneurs hépatiques en triglycérides, ARNₘ d'apo B et apo B sont indiqués dans les tableaux 9, 10 et 11.

**TABLEAU 8**

| Teneurs en métabolites sanguins en 2ème et 4ème semaines de lactation (1) | | | | |
|---|---|---|---|---|
| | | Témoin (n = 6) | Méthionine (n = 5) | Méth. + Lysine (N = 5) |
| AGNE (mM) | S2 | 0,68 (± 0,15) | 0,66 (± 0,14) | 0,47 (± 0,15) |
| | S4 | 0,48 (± 0,11) | 0,45 (± 0,11) | 0,33 (± 0,11) |
| Glucose (mg/dl ) | S2 | 37 (± 3 ) | 42 (± 4) | 42 (± 4) |
| | S4 | 40 (± 4) | 36 (± 4) | 40 (± 4) |
| Lactate (mM) | S2 | 0,54 (± 0,13) | 0,79 (± 0,14) | 0,88 (± 0,14)^{a} |
| | S4 | 0,85 (± 0,18 ) | 0,63 (± 0,20) | 0,56 (± 0,20) |
| β-hydroxybutyrate (mM) | S2 | 2,78 (±0,42) | 1,86 (± 0,45)^{b} | 2,26 (± 0,50) |
| | S4 | 3,01 (± 0,70) | 2,93 (± 0,75) | 2,93 (± 0,83) |
| Acétone (mg/dl) | S2 | 6,1 (±1,1) | 2,2 (±1,3)^{c} | 3,0 (± 1,3)^{d} |
| | Si | 5,7 (± 2,7) | 4,6 (± 2,9) | 5,9 (± 3,0) |
| Urée (mg/dl) | S2 | 37,1 (± 3,3 ) | 36,5 (± 4,4) | 28,5 (± 3,5)^{e} |
| | S4 | 39,2 (± 3,4) | 37,4 (± 4,6) | 32,7 (± 3,6) |

| | | | | |
|---|---|---|---|---|
| (1) Valeurs ajustées en fonction de la covariable de première semaine : a Différent du lot témoin (P < 0,10) | | | | |
| b. Différent du lot témoin (P < 0,17) | | | | |
| c. Différent du lot témoin (P < 0,05) | | | | |
| d. Différent du lot témoin (P < 0,11) | | | | |
| e Différent du lot témoin (P < 0,11) | | | | |

**TABLEAU 9**

| Teneurs hépatiques en triglycérides (mg/g foie frais congelé) | | | |
|---|---|---|---|
| Semaines après vêlage | S1 | S2 | S4 |
| Témoin (n = 6) | 54,5 ±12,5 | 70,6 ±19,9 | 55,2 ± 15,3 |
| Méthionine (n = 5) | 34,6 ± 4,1^{a} | 67,6±11,8^{b} | 36,4 ± 9,7^{a} |
| Mét. + lys. (n = 5) | 36,2 ± 5,9 | 46,2 ± 13,2 | 25,3 ± 9,8 |
| a, b : P < 0,05. Test t de comparaison de moyennes d'une même ligne dans le cas de séries appariées. | | | |

La méthionine et/ou la lysine ont été distribuées après la semaine S1.

**TABLEAU 10**

| Teneurs hépatiques en ARNm d'apo B (U.A./µg ARN totaux) | | | |
|---|---|---|---|
| Semaines après vêlage | S1 | S2 | S4 |
| Témoin (n = 6) | 134,8 ± 20,7 | 150,8 ± 21,9 | 130,1 ± 17,4 |
| Méthionine (n = 5) | 101,3 ± 18,4^{a} | 83,5 ± 11,4^{a} | 151,6 ± 30,1^{b} |
| Mét. + lys. (n = 5) | 97,9 ± 118,3^{c} | 155,9 ± 19,2^{a} | 123,2 ± 16,4^{bc} |
| a, b : P < 0,05 ; a, c : P < 0,10. Test t de comparaison de moyennes d'une même ligne dans le cas de séries appariées. | | | |

La méthionine et/ou la lysine ont été distribuées après la semaine S1.

**TABLEAU 11**

| Teneurs hépatique en apo B (U. A/10⁹ cellules) | | | |
|---|---|---|---|
| Semaines après vêlage | S1 | S2 | S4 |
| Témoin (n = 6) | 53,94 ± 6,25 | 50,6 ± 7,2 | 66,4 ± 17,8 |
| Méthionine (n = 5) | 45,5 ± 8,1^{a} | 41,4 ± 12,1^{a} | 65,6 ± 12,6^{b} |
| Mét. + lys. (n = 5) | 46,2 ± 7,7^{c} | 65,7 ± 9,6^{d} | 99,8 ± 41,2^{d} |
| a, b : P < 0,03 ; c, d : P < 0,2. Test t de comparaison de moyennes d'une même ligne dans le cas de séries appariées | | | |

La méthionine et/ou la lysine ont été distribuées après la semaine S1.

Les teneurs en métabolites sanguins (tableau 8) ne montrent que peu d'effets significatifs des traitements. On observe toutefois qu'en deuxième semaine de lactation, le traitement par la méthionine a diminué significativement les teneurs en corps cétoniques (β.hydroxybutyrate et acétone). Des tendances similaires, mais moins significatives, sont observées avec le traitement méthionine + lysine

Le lot Témoin présente des teneurs hépatiques en triglycérides (TG) assez élevées en semaine S1 (première semaine après vêlage) qui tendent à augmenter en semaine S2 (+ 30 %, NS) puis à diminuer légèrement de la même amplitude en semaine S4 (- 22 %, NS). Les vaches étaient au début de lactation en état de stéatose (54,5 - 70,6 mg de TG/g de foie)

Dans le lot supplémenté en méthionine, les teneurs hépatiques en TG sont plus faibles en semaine S1 (avant traitement par la méthionine) que dans le lot Témoin mais augmentent fortement en semaine S2 (+ 95 %, P < 0,05) pour atteindre des valeurs comparables à celles observées dans le lot Témoin. Toutefois, l'infiltration lipidique diminue de façon significative en semaine S4 (- 46 %, P < 0,05). Une augmentation significative a la fois des niveaux intrahépatiques de l'ARNm d'apo B (+ 82 %, P < 0,05) et de l'apo B (+ 58 %, P < 0,03) apparaît en semaine 4 par rapport à la semaine S2.

Dans le lot supplémenté en méthionine et en lysine, les teneurs hépatiques en TG en semaine S1 (avant traitement par la méthionine + lysine) sont comparables à celles observées pour le lot supplémenté en méthionine. Toutefois, on n'observe pas d'augmentation importante du taux en TG en semaine S2, contrairement au lot supplémenté en méthionine seule. La baisse entre les semaines S2 et S4 conduit à un faible taux d'infiltration lipidique en semaine S4. Une augmentation des niveaux intrahépatiques de l'ARNm d'apo B (+ 59 %, P < 0,10) et de l'apo B (+ 42 %, P < 0,20) apparaît dès la semaine S2 pour toutes les vaches malgré un écart type important.

## Revendications

1. Utilisation de méthionine et/ou lysine, non associé à de la choline, protégé vis à vis du rumen pour produire une composition destinée à être administrée par voie orale aux ruminants en vue de prévenir ou traiter la stéatose hépatique.

2. Utilisation selon la revendication 1 en vue de diminuer la teneur en triglycérides au niveau hépatique.

3. Utilisation selon la revendication 1 pour augmenter la synthèse de l'apoprotéine B hépatique.

4. Utilisation selon la revendication 1 caractérisée en ce que la méthionine et/ou lysine est protégée par un mélange à base d'un polymère pH sensible et d'un corps gras.

5. Utilisation selon la revendication 5 caractérisée en ce que la méthionine et/ou lysine est protégée par un mélange à base d'un copolymère vinylpyridine styrène et d'un acide gras.

6. Utilisation selon la revendication 1, caractérisé en ce que la méthionine et/ou lysine est protégée par un polymère naturel à base de chitosane.

7. Utilisation selon la revendication 1 caractérisée en ce que la méthionine et/ou lysine est protégée par un polymère naturel dont la libération est provoquée par une hydrolyse enzymatique.

8. Utilisation selon la revendication 8 caractérisée en ce que la méthionine et/ou lysine est protégée par un polymère à base de zéine.

## Patentansprüche

1. Verwendung von nicht mit Cholin assoziiertem Methionin und/oder Lysin, das gegen den Pansen geschützt ist, zur Herstellung einer Zusammensetzung zur oralen Verabreichung an Wiederkäuer, um Fettleber zu verhindern oder zu behandeln.

2. Verwendung nach Anspruch 1, zur Senkung des Gehalts an Triglyceriden in der Leber.

3. Verwendung nach Anspruch 1, zur Erhöhung der Synthese von Leber-Apoprotein B

4. Verwendung nach Anspruch 1, dadurch gekennzeichnet, daß das Methionin und/oder Lysin durch ein Gemisch auf der Grundlage eines pH-empfindlichen Polymeren und eines Fettkörpers geschützt ist.

5. Verwendung nach Anspruch 4, dadurch gekennzeichnet, daß das Methionin und/oder Lysin durch ein Gemisch auf der Grundlage eines Vinylpyridin-Styrol-Copolymeren und einer Fettsäure geschützt ist.

6. Verwendung nach Anspruch 1, dadurch gekennzeichnet, daß das Methionin und/oder Lysin durch ein natürliches Polymeres auf der Basis von Chitosan geschützt ist.

7. Verwendung nach Anspruch 1, dadurch gekennzeichnet, daß das Methionin und/oder Lysin durch ein natürliches Polymeres geschützt ist, dessen Freisetzung durch eine enzymatische Hydrolyse hervorgerufen wird.

8. Verwendung nach Anspruch 7, dadurch gekennzeichnet, daß das Methionin und/oder Lysin durch ein Polymeres auf der Basis von Zein geschützt ist.

## Claims

1. Use of methionine and/or lysine, not combined with choline, protected from the rumen, for producing a composition intended to be administered orally to ruminants for preventing or treating hepatic steatosis.

2. Use according to Claim 1, for reducing the content of triglycerides at the hepatic level.

3. Use according to Claim 1, for increasing the synthesis of hepatic apoprotein B.

4. Use according to Claim 1, characterized in that the methionine and/or lysine is protected by a mixture based on a pH-sensitive polymer and a fatty substance.

5. Use according to Claim 4, characterized in that the methionine and/or lysine is protected by a mixture based on a vinylpyridine/styrene copolymer and a fatty acid.

6. use according to Claim 1, characterized in that the methionine and/or lysine is protected by a natural polymer based on chitosan.

7. Use according to Claim 1, characterized in that the methionine and/or lysine is protected by a natural polymer whose release is caused by an enzymatic hydrolysis.

8. Use according to Claim 7, characterized in that the methionine and/or lysine is protected by a polymer based on zein.
